# EUROPÄISCHE PATENTSCHRIFT

(11) **EP 0 959 849 B1**
(45) Veröffentlichungstag und Bekanntmachung des Hinweises auf die Patenterteilung: **21.01.2004**
(21) Anmeldenummer: 97903242.2
(22) Anmeldetag: 10.02.1997
(51) Int. Cl.: A61F 13/26, A61F 13/15

(54) **HYGIENISCHER BENUTZERSCHUTZ**
HYGIENIC USER PROTECTION
PROTECTION HYGIENIQUE

(30) Priorität: 13.02.1996 DE 19606902
(43) Veröffentlichungstag der Anmeldung: 01.12.1999
(73) Patentinhaber: Wagner, Maria, 12679 Berlin (DE)
(72) Erfinder: WAGNER, Maria, D-12679 Berlin (DE); MINNINGER, Konrad, D-12679 Berlin (DE)
(86) Internationale Anmeldenummer: PCT/EP1997/000576
(87) Internationale Veröffentlichungsnummer: WO 1997/029724

(56) Entgegenhaltungen:
- EP-A- 0 237 782
- EP-A- 0 347 319
- CH-A- 540 689
- DE-A- 1 915 192
- US-A- 2 693 806
- US-A- 3 749 093
- US-A- 3 973 567

## Beschreibung

### Technisches Gebiet

Die Erfindung betrifft Damen-Hygiene-Artikel, wie sie heute weltweit gebräuchlich sind.
Diese Hygiene-Artikel finden naturgemäß verstärkt Anwendung während der Menstruation, jedoch sind seit Jahren auch Hygiene-Einlagen für den täglichen Gebrauch allgemein üblich und finden immer häufiger Einsatz.
Solche Hygiene-Artikel sind landläufig als "Damen-Binden", "Slip-Einlagen" bekannt. Hygiene-Artikel betreffend werden hohe Anforderungen an Keimfreiheit, sterile Herstellung und Verpackung gestellt.

### Zugrundeliegender Stand der Technik

Solche bekannten Hygiene-Artikel finden in zunehmendem Maße Anwendung, besitzen jedoch keine Deckschicht, keinen Anwendungsschutz, in Folge kurz Deckschicht oder "GHA" bezeichnet, welcher die Körperkontaktfläche des Hygiene-Artikel, bis unmittelbar vor Gebrauch, vor Funktionsaufnahme, vor Kontaminierung, Verkeimung schützt. Aus EP-A-0,347,319 ist eine Deckschicht als Einzelverpackung bekannt, die entsprechende Hygieneprodukte umhüllt, welche nur teilweise trennbar angebracht ist und auch nur teilweise von der Oberseite her abgelöst werden kann;
der erfindungsgemäßen Lösung lag unter anderem die Aufgabe zugrunde, einen umfassenden Schutz gegen Verunreinigungen der Körperkontaktfläche von Damenhygieneartikeln insbesondere auch während der Handhabung, vor und bei Ingebrauchnahme derart zu gestalten, dass der erfindungsgemäße Gegenstand einerseits die Körperkontaktfläche vollkommen zuverlässig abdeckt und andererseits vollständig davon trennbar ist. Kein nach dem Stand der Technik bekanntes Dokument, auch nicht durch US-A-2,693,806 und/oder US-A-3,973,567 entspricht den kennzeichnenden Teilen der erfindungsgemäßen Lösung - in keinem der vorliegenden, bekannten Dokumente wird eine Deckschicht beschrieben oder auch nur nahegelegt, deren Deckschicht unterseitig derart gestaltet ist, dass diese Deckschicht - von der Körperkontaktfläche, der eingangs beschriebenen Hygieneartikel abgetrennt werden kann.

### Offenbarung der Erfindung

Der Ausgangspunkt zur Erfindung ist ein wesentlich erhöhtes Bedürfnis nach nicht kontaminierbaren Anwendungsoberflächen von Hygiene-Artikeln, welche direktem Körperkontakt ausgesetzt sind, aufgrund zunehmender Infektions-Übertragungsgefahr.

Alle derzeit im Gebrauch befindlichen "Einlagen". .Binden" o.a. wie abgewandelte Formen und Arten solcher Artikel werden vor Funktionsaufnahme von Hand angefasst, die Körperkontaktfläche des Hygiene-Artikels berührt, bei Artikeln mit rückseitiger Haftklebefläche durch Andrücken auf die Haltefläche, Slip oder ähnliches, zusätzlich kontaminiert, wodurch sich zwangsläufig, je nach Ort wie Art der Anwendung bzw. Anbringung, hohe und höchste Gefahr von direkten wie indirekten Verkeimungen - ein gesteigertes Infektionsrisiko - ergibt.

Um Kontamination weitestgehend auszuschließen, nahezu unmöglich zu machen, musste eine Deckschicht, ein Anwendungsschutz erfunden, die Handhabung dieses Schutzes zudem einfach, sicher in der Handhabung, bei kostengünstiger Herstellung, gestaltet werden.

Keiner der sich derzeit im Gebrauch befindlichen Damen-Hygiene-Artikel o. ä. besitzt eine Deckschicht, einen Anwendungsschutz, Kontaminationsschutz zur Vermeidung von Verkeimungen.
Im Gegenteil, alle diese Artikel werden zum Gebrauch vom Anwender, in Krankenhäusern oder ähnlichen Einrichtungen wie auch Altenpflege usw. teilweise sogar von mehreren Personen angefasst und zwangsläufig kontaminiert.

Der Erfindung lag die Aufgabe zugrunde, eine Deckschicht, einen Anwendungsschutz für Hygiene-Artikel vorgenannter Art, in häufigem Maße auch bei Inkontinenz Gebrauch findend, zu entwickeln und zu schaffen, welcher nach seinem Entfernen, unmittelbar vor Funktionsaufnahme des Hygiene-Artikels, höchste Ansprüche an Sauberkeit und Keimfreiheit der Körperkontaktflächen des Hygiene-Körpers bei Funktionsaufnahme gewährleistet, Eigen- oder Fremdkontamination vor und/oder unmittelbar bei der Handhabung zur Anwendung vorgenannter Hygiene-Artikel - bei sachgemäßer Anwendung - weitestgehend ausschließt, nahezu unmöglich macht.
Die Ausführung der Erfindung ist industriell machbar und kann durch standardisierte Massenfertigung kostengünstig gestaltet werden.
Der erfindungsgemäße Gegenstand kann bei einfachster Handhabung wirkungsvoll eingesetzt werden.
Durch die erfindungsgemäße Art und Form der Deckschicht ist nur ein Minimum an technischem Aufwand erforderlich.
Die Herstellungskosten sind gering, bei höchster Effizienz.
Die Akzeptanz von Anwendern wird wesentlich durch die gewählte Ausführung gefördert, zumal das subjektive Schutzbedürfnis des Einzelnen, wie das der sich in Verantwortung Befindenden - bereits vorhanden - fortschreibend zunimmt.

Ein- oder Mehrfachinfektionen - durch Benutzung von Gemeinschaftsanlagen, Kontaminationsrisiko im Genitalbereich allgemein - welche sehr oft sehr spät, zu spät erkannt, weil auch unterschätzt, erst nach längerer Inkubationszeit offensichtlich, jedoch hohe Risiken für die Gesundheit des Einzelnen bergend und mit enormen Medikamenten- und Heilbedarf verbunden, zusätzlich oft langwierige weitere Ansteckungs- und Krankheitsfolgen hervorrufend, hohe Kosten bereitend, wird wirkungsvoll vorgebeugt.
Sogenannte "harmlose" wie auch schwerwiegende behandlungshedürftige Infektionen von Anwendern, durch Eigen- wie Fremdkontamination der Körperkontaktfläche des Hygiene-Körpers werden nahezu ausgeschlossen, unmöglich.

Die Erfindung besitzt zudem hohen volkswirtschaftlichen Wert. dient in großem Maße zur Eindämmung von Infektionen, bewahrt Anwender vor Schaden und Kosten; die Volkswirtschaft im allgemeinen vor einem hohen Anteil an Krankheitsfolgen, Kostenund Folgekosten.
Der Erfindung lag die Aufgabe zugrunde, einen Hygiene-. Damen-Hygiene-Artikel in Gesamtheit zu schaffen, welcher bei sachgemäßer Anwendung, vor und bei Handhabung Kontaminationen von Körperkontaktflächen bei Hygiene-Artikeln, insbesondere solcher im Genitalbereich eingesetzter Hygiene-Körper, insbesondere im weiblichen Genitalbereich, verhindert bzw. nahezu unmöglich macht.

Erfindungsgemäß wurde diese Aufgabe dadurch gelöst, dass Anwendungs- oder Gebrauchsartikel, speziell Hygiene-Artikel, insbesondere Damen-Hygiene-Artikel wie Damen-Binden, Slip-Einlagen, o. ä. mit einer Deckschicht, Anwendungsschutz direkt entweder hergestellt oder mit einer solchen nachträglich versehen werden.

Die erfindungsgemäße Lösung der Aufgabe beinhaltet einfachste, industriell machbare Herstellung wie evtl. Nachrüstung von bereits existierenden Formen und Arten solcher Hygiene-Artikel, insbesondere von Damen-Hygiene-Artikeln.
Größtmögliche Akzeptanz musste sichergestellt, einfachste Ver- und Anwendung gewährleistet, eine Kontaminierung entweder durch den Anwender wie auch Hilfs- oder Drittpersonen, nahezu unmöglich gemacht werden, der erfindungsgemäße Gegenstand löst alle gestellten Auflagen in optimal erreichbarster Form, Art und Weise.

Die erfindungsgemäße Lösung stellt auch bei Verwendung an besonders kritischen Orten - wie u.a. öffentlichen Toilettenanlagen, bei Nutzung zwangsgebundener Hygiene-Einrichtungen wie im häuslichen Bereich selbst, bei allgemein benutzten Einrichtungen wie öffentlichen Toiletten, Bahn-, Bus-, Flugzeugtoiletten, Gaststätten-, Hotel-, Versorgungseinrichtungs-Toiletten, nicht zuletzt und ohne den Anspruch auf Vollständigkeit sei darauf hingewiesen, auch im Falle von Notdurft sicher, dass bei Gebrauch von Hygieneartikeln mit einer Deckschicht eine Verkeimung der angewendeten Hygiene-Artikel nahezu unmöglich wird.
Die erfindungsgemäße Lösung, der erfindungsgemäße Gegenstand, erfindungsgemäß hergestellte Hygiene-Artikel, insbesondere Damen Hygiene-Artikel hilft, das - zwangsläufig natürlich - hohe. ( derzeit noch allgemein als gegeben angesehene ) Kontaminationsrisiko durch Eigen- insbesondere Fremdkontamination innerhalb von Pflege- wie Krankenversorgungseinrichtungen, ja Krankenhäusern selbst, für Personal, zu Betreuende wie Verwender insgesamt, auf ein Minimum zu reduzieren.
Der erfindungsgemäße Gegenstand, die Deckschicht, der Anwendungsschutz wird am Hygiene-Artikel an oder aufgebracht, hinzugefügt, direkt oder nachträglich befestigt oder geheftet oder sonstwie leichtentfemlich damit verbunden, ist mehr oder weniger flexibel und von einer Stärke welche vom Mikrobereich an, bis hin zu mm oder mehr betragen kann.
Die Deckschicht ist eine ein- oder mehrteilige Abdeckung und wird zusätzlich zum Hygiene-Körper hinzugefügt oder angebracht, kann aus unterschiedlichsten Materialien unterschiedlichster Größe hergestellt werden, ist in vielfältigsten Formen und Arten unterschiedlichster Art und Weise herstellbar und kann auch als Ein-, Mehr- oder Vielfach-, Primär-, Sekundär-, Mehrfachverpackung dienen.
Diese Deckschicht oder Hinzufügung/en ist/sind in Gesamtheit vollumfänglich zu schützen.

| **Zeichnungen/Figurenbeschreibungen/Legende** | | |
|---|---|---|
| **Deckschicht/** | | |
| Hygiene Anwendungsschutz | | **I** |
| Hygiene-Artikel | Damenbinde | |
| | Slip- Einlage | **II** |
| Haftklebeabdeckung | | **III** |
| | | |
| Figur 1 a) Damen-Hygieneartikel | Damenbinde, Slipeinlage Einzelbestandteile | - Draufsicht- |
| | | |
| Figur 1b) Damen-Hygieneartikel | Damenbinde. Slipeinlage | - Draufsicht- |
| | | - komplett |
| | | |
| Figur 1 c) Damen-Hygieneartikel | Damenbinde, Slipeinlage | - Einzelbestandteile |
| | | |
| | | - Seitenansicht |

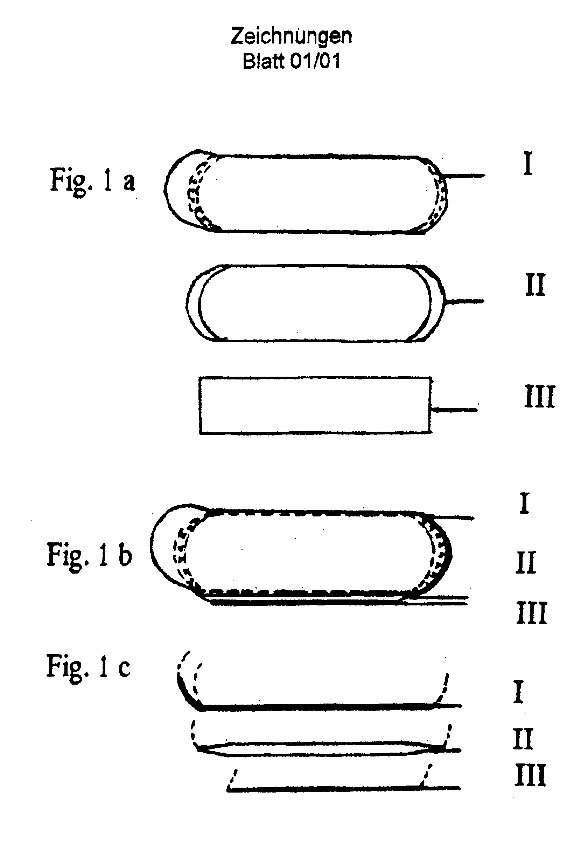

## Patentansprüche

1. ***Deckschicht* [(Top Sheet) Gebrauchs- Hygiene-Anwendungsschutz kurz "GHA" zur Abdeckung von Körperkontaktflächen von Hygiene Artikeln, äußerlich zur Anwendung kommenden Einlagen, Hygiene-Artikeln des Genitalbereiches, Damen-Hygiene-Artikeln ( z.B. Binden, Slip-/Einlagen; Damenbinden ) *dadurch gekennzeichnet, dass*** die Deckschicht die Körperkontaktfläche des Artikels ganz, ein- oder mehrteilig, ein- oder mehrlagig bedeckt, und/oder den Artikel teilweise oder vollkommen umhüllt und dass die Unterseite der Deckschicht so gestaltet ist, dass die Deckschicht mit der Oberseite des Hygieneartikels oder Teilen davon verbunden, verklebt, geheftet oder in sonstiger Weise verbunden - adhäsiv und/oder kohäsiv -, ablösbar zu- oder aneinandergefügt ist/sind und mit dem Hygiene-Artikel vollständig trennbar verbunden ist.

2. ***Deckschicht nach Anspruch 1), dadurch gekennzeichnet, dass*** diese Deckschicht aus Papier, Pappe, Baumwolle, Zellstoff, Kunstoffmaterialien, Natur- wie Kunstfasern im Allgemeinen als erst- wie wiederverarbeitetes Material, aus vorgenannten wie evtl. Ersatzstoffen, wie Mischprodukten aus vorgenannten wie sonstigen, geeigneten Ersatzstoffen besteht.

3. ***Deckschicht nach Anspruch 1) bis 3), dadurch gekennzeichnet, dass*** diese Deckschicht mit einer oder mehreren Abzugs-/ Entfernungslaschen versehen ist und diese Teil der Deckschicht selbst oder integrierter oder hinzugefügter Bestandteil nach Ansprüchen 1) und 2) sind.

4. ***Deckschicht nach Anspruch 1) bis 3), dadurch gekennzeichnet, dass*** diese Deckschicht durch Press-, Punkthaftung. Presspunkthaftung, wie von Papier bzw. Zellstofftaschentüchern bekannt, insbesondere bei Damen-Binden. Slipeinlagen ein-, mehr- oder allseitig befestigt ist.

## Claims

1. ***Top Sheet, medical application Cover, called for short maC for covering of physical contact surfaces of hygiene articles for external application only coming in use liners, hygiene articles of the genital sphere, ladies' hygiene articles (for example panty liners; napkins) characterized that way, that*** the top sheet covers the physical contact surface of the article completely, in one or some parts, in one or several layers and that the underside of the top sheet is designed this way, that the top sheet is connected with the upperside of the hygiene article or parts of it differently sticked or fixed to it, or in another way connected with it - are adhesively and / or cohesively - removably added one to the other and is completely separably connected with the hygiene article.

2. ***Top Sheet according to claim 1. ) is characterized that way, that*** this top sheet consists of paper, cardboard, cotton, cellulose, synthetic materials, natural like synthetic fibers generally as first manufactured or recycled material of previously named or possibily of substitute materials, like mixed products out of previously named like other suitable substitute materials.

3. ***Top Sheet according to claim 1. ) and 2.) is characteristiced that way, that*** this top sheet is furnished with one or some flaps to stripp it off, and these flaps are integrated or added part of the top Sheet according to the claims 1. ) and 2.).

4. ***Top Sheet according to the claims 1. ) to 3.) is characteristized that way, that*** this top sheet is fixed by presspoint, presspoint clinging, as it's wellknown by tissues and especially will be fixed at napkins, by napkins, liners from one, or from all sides.

## Revendications

1. ***Couche de couvrir, d'utilisation protection d'application d'hygiène brièvement "GHA ", pour la couverture des surfaces de contact de corps del l'hygiène les articles extérieurement pour appliquer des insertions prochaines, les articles d'hygiène du Domaine genital, les articles pour l'hygiène féminine, ( par exemple protection periodique, les serviettes hygiénique, recouvrements panty, protège-slip ), charactérisée en ce que, celle la Couche de couvrir le domaine*** de contact physique de l'article complètement, dedans ou de liasse multiple, et/ou qui des articles en partie ou parfaitement enveloppés et ce le dessous de la Couche de couvrir est ainsi conçu, que la Couche de couvrir attachée collé ou elle reliée dans une autre maniére relié à la haute page de l'article d'hygiene ou à des parties de cela, sticked ou a fixé à lui, sont reliés d'une manière ou d'une autre additionné démontable - l'adhésif et/ou cohésif - à autre est relié à l'article d'hygiène complètement séparable.

2. ***Couche de couvrir, selon la condition 1. ), par le fait caractérisée que cette*** couche de couvrir compose en general du carton, du papier, du coton, de la cellulose, art outre des matériaux, nature telle que les fibres artificielles généralement aussi d'abord comme le matériel encore-fini, de mentionné ci-dessus que l'evtl. Produits de remplacement, comme consiste produits de mélange de mentionné ci-dessus comme autre, produits de remplacement appropriés.

3. ***Couche de couvrir, selon la condition 1. ) jusqu'à ce que 2. ), par le fait caractérisée cette*** couche de couvrire est équipée de ce fait d'un ou plusieurs verrous de distance de départ, et la présente partie de la couche de couvrire ou d'un composant intégré ou supplémentaire selon les conditions 1. ) jusqu'à ce que 2. ).

4. ***Couche de couvrir, selon la condition 1. ) jusgu'à ce que 3. ), par le fait ca ractérisée que cette*** couche de couvrire par la pression, adhérence de point. Serrez l'adhérence de point, comme des mouchoirs de papier et/ou de cellulose admet, particulierèment soient fixes aux articles d'hygiène de dames, serviettes, recouvrements panty d'un, plus ou est généralement attachées.
